(19) **Europäisches Patentamt / European Patent Office / Office européen des brevets**

(11) **EP 1 839 570 A2**

(12) **EUROPEAN PATENT APPLICATION**

(43) Date of publication:
**03.10.2007 Bulletin 2007/40**

(51) Int Cl.:
**A61B 5/00** *(2006.01)*

(21) Application number: **07006537.0**

(22) Date of filing: **29.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **30.03.2006 JP 2006092708**

(71) Applicant: **SYSMEX CORPORATION**
**Kobe-shi,**
**Hyogo 651-0073 (JP)**

(72) Inventors:
• **Maekawa, Yasunori**
  **Kobe-shi**
  **Hyogo 651-0073 (JP)**
• **Sato, Toshiyuki**
  **Kobe-shi**
  **Hyogo 651-0073 (JP)**

• **Sawa, Kenichi**
  **Kobe-shi**
  **Hyogo 651-0073 (JP)**
• **Okada, Seiki**
  **Kobe-shi**
  **Hyogo 651-0073 (JP)**
• **Hagino, Kei**
  **Kobe-shi**
  **Hyogo 651-0073 (JP)**
• **Asakura, Yoshihiro**
  **Kobe-shi**
  **Hyogo 651-0073 (JP)**
• **Ohnishi, Yasuhito**
  **Kobe-shi**
  **Hyogo 651-0073 (JP)**

(74) Representative: **HOFFMANN EITLE**
**Patent- und Rechtsanwälte**
**Arabellastrasse 4**
**81925 München (DE)**

(54) **Analyzer and analyzing method**

(57) The present invention is to present an analyzer for analyzing a concentration of a predetermined component contained in tissue fluid of a subject, the analyzer being capable of mitigating the pain of the subject. The analyzer comprises: an extraction medium retainer for retaining an extraction medium for holding tissue fluid extracted through a skin of a subject; a component amount information obtainer for obtaining component amount information regarding amount of a predetermined component contained in the tissue fluid held in the extraction medium; an electrical information obtainer for obtaining electrical information related to amount of the extracted tissue fluid by supplying electrical power to the extraction medium which is holding the extracted tissue fluid; and a component concentration obtainer for obtaining a concentration of the predetermined component contained in body fluid of the subject based on the component amount information and the electrical information.

FIG. 3

## Description

FIELD OF THE INVENTION

The present invention relates to a device and a method for measuring a concentration of a predetermined component contained in body fluid of a subject.

BACKGROUND

A method for measuring blood collected from a finger via a lancet mechanism using blood glucose test paper is disclosed as a blood glucose measuring method in US Patent No. 6,607,543. Furthermore, a device for executing this method is also commercially available. This device, however, is painful to the subject because blood is collected by piercing the finger of the subject.

A glucose extraction method using reverse iontophoresis for transdermally extracting glucose by applying electrical energy to the skin is disclosed as a method for mitigating the pain of the subject in WO 96/00110.

In order to confirm the correlation between amount of the extracted glucose and blood glucose level, however, blood must actually be collected to measure blood glucose level in the method disclosed in WO 96/00110. Therefore, the subject inevitably experiences pain due to blood collection.

SUMMARY

The first aspect of the present invention is an analyzer, comprising: an extraction medium retainer for retaining an extraction medium for holding tissue fluid extracted through a skin of a subject; a component amount information obtainer for obtaining component amount information regarding amount of a predetermined component contained in the tissue fluid held in the extraction medium; an electrical information obtainer for obtaining electrical information related to amount of the extracted tissue fluid by supplying electrical power to the extraction medium which is holding the extracted tissue fluid; and a component concentration obtainer for obtaining a concentration of the predetermined component contained in body fluid of the subject based on the component amount information and the electrical information.

The second aspect of the present invention is an analyzing method, comprising steps of (a) extracting tissue fluid through the skin of a subject into an extraction medium for holding the extracted tissue fluid; (b) obtaining component amount information regarding amount of a predetermined component contained in the tissue fluid held in the extraction medium; (c) obtaining electrical information related to amount of the extracted tissue fluid by supplying electrical power to the extraction medium which is holding the extracted tissue fluid; and (d) obtaining a concentration of the predetermined component contained in body fluid of the subject based on the component amount information and the electrical information.

BRIEF DESCRIPTION OF THE DRAWINGS

Fig. 1 is a perspective view of a blood glucose level measuring device 1 mounted on the wrist of a subject;
Fig. 2 is a perspective view of a band 19 provided with a fixture 20;
Fig. 3 shows the internal structure of the blood glucose level measuring device 1 installed with an extraction cartridge 2;
Fig. 4 is a structural diagram showing the structure of the blood glucose level measuring device 1 of Fig. 3;
Fig. 5 is a top view showing the structure of the extraction cartridge 2;
Fig. 6 is a cross section view along the VI-VI line of the extraction cartridge 2 shown in Fig. 5;
Fig. 7 is a perspective view of the extraction cartridge set 200;
Fig. 8 is a cross section view along the VIII-VIII line of the extraction cartridge set 200 shown in Fig. 7;
Fig. 9 is a perspective view of the extraction cartridge set 200;
Fig. 10 is a perspective view showing the micro needle array 51 used in a preparatory step;
Fig. 11 shows the structure of the detector 30 for detecting glucose by illuminating a glucose sensor 70;
Fig. 12 is a flow chart showing preparation sequence of the subject for measurement by the blood glucose level measuring device 1;
Fig. 13 is a flow chart showing the measurement operation sequence of the controller-analyzer 11 of the blood glucose level measuring device 1;
Fig. 14 to 16 and Fig. 18 are schematic views illustrating the glucose extraction principle with the use of the blood glucose level measuring device 1;
Fig. 17 shows the electrical circuit when an electric field is applied to the skin of the subject;
Fig. 19 shows the electrical circuit when measuring the electrical conductance of the extraction medium; and
Fig. 20 is a characteristics chart showing the relationship between the electrical conductance of the extraction medium and the glucose extraction speed.

DETAILED DESCRIPTION OF THE PREFERRED EMBODIMENT

[General Structure of Blood glucose Level Measuring Device 1]

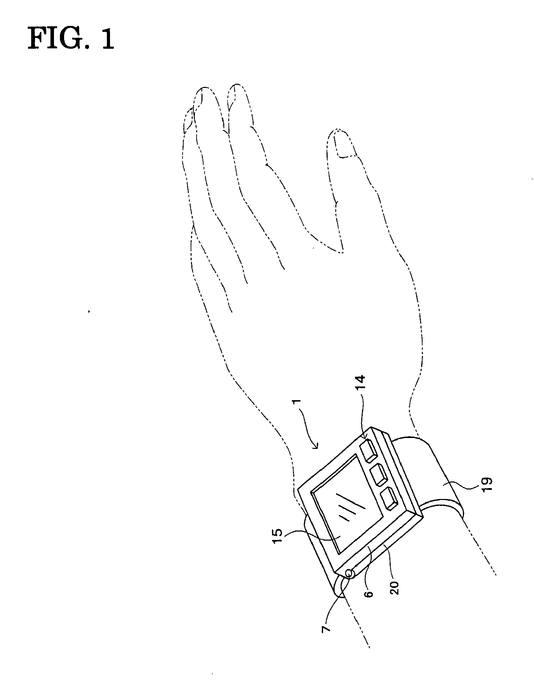

The embodiments of the present invention are described hereinafter based on the drawings.
The general structure of the blood glucose level measuring device 1 including an installed extraction cartridge 2 is first described using Figs. 1 through 4. The embodiment of the blood glucose level measuring device 1 of the present invention is a device for extracting tissue fluid containing glucose as a biochemical component from a body, and calculating blood glucose level by analyzing the glucose contained in the extracted tissue fluid. The tissue fluid is the intercellular fluid component of the tissue of animals.

The subjects are mainly homo sapiens.

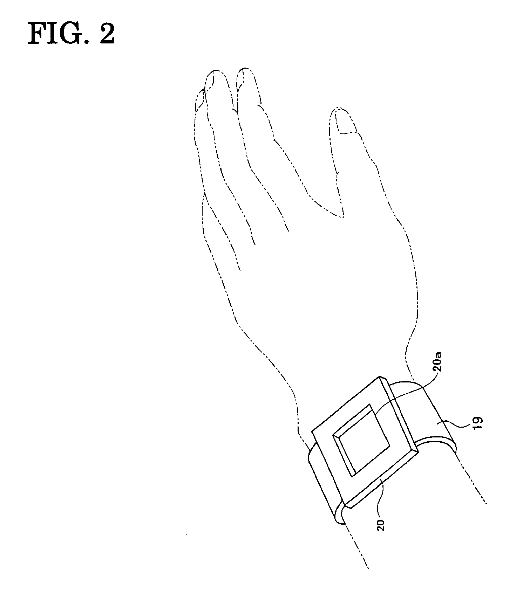

As shown in Fig. 1, the blood glucose level measuring device 1 is configured so as to be mountable on the wrist of a subject using the band 19. The blood glucose level measuring device 1 is provided with a hinge 7 that joins the analyzing unit 6 and fixture 20, and the analyzing unit 6 is disposed so as to contact with the surface of the fixture 20 via the opening and closing operation of the hinge 7. Fig. 2 shows the band 19 when the analyzing unit 6 is removed from the blood glucose level measuring device 1. As shown in Fig. 2, the fixture 20 of the band 19 is provided with an opening 20a at a predetermined position, and the opening 20a is configured such that the skin of the subject is exposed through the fixture 20. Furthermore, an electrode 3 (not shown in the drawing) is attached to the back surface of the band 19. A gold electrode is used as the electrode 3.

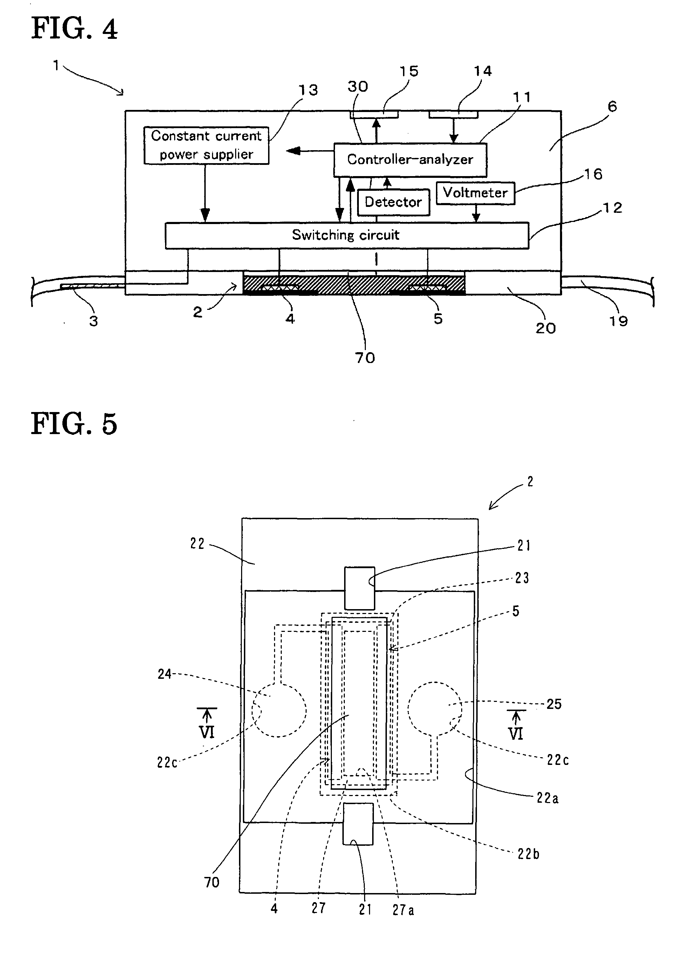

The blood glucose level measuring device 1 is provided with the analyzing unit 6, extraction cartridge 2 removably fixed on the analyzing unit 6, and the band 19, as shown in Fig. 4. When the analyzing unit 6 with the fixed extraction cartridge 2 is installed on the fixture 20 of the band 19, the extraction cartridge 2 fixed on the analyzing unit 6 contacts with the skin of the subject through the opening 20a of the fixture 20.

As shown in Fig. 4, the analyzing unit 6 is provided with a controller-analyzer 11, switching circuit 12, input part 14, display (LCD) 15, direct current type constant current power supplier 13, voltmeter 16 for measuring the voltage supplied from the constant current power supplier 13 and outputting the voltage level to the controller-analyzer 11, and a detector 30. The analyzing unit 6 is installed on the fixture 20 of the band 19 and used to measure a blood glucose level.

The controller-analyzer 11 is provided to control the output of the constant current power supplier 13 and the operation of the switching circuit 12, and receive the output of the detector 30 and output of the voltmeter 16, and calculate the blood glucose level. The controller-analyzer 11 is configured by a micro computer that includes a CPU, ROM, RAM and the like.

The display (LCD) 15 is provided to display the calculation result (for example, blood glucose level) of the controller-analyzer 11.

The input part 14 is provided for inputting a measurement start command and necessary measuring conditions to the controller-analyzer 11.

The constant current power supplier 13 is provided for supplying a voltage between the electrode 3 provided on the band 19, the electrode 4 and electrode 5 provided on the extraction cartridge 2. The constant current power supplier 13 is configured by combining a transistor constant current circuit and a transistor constant voltage circuit with an internal battery.

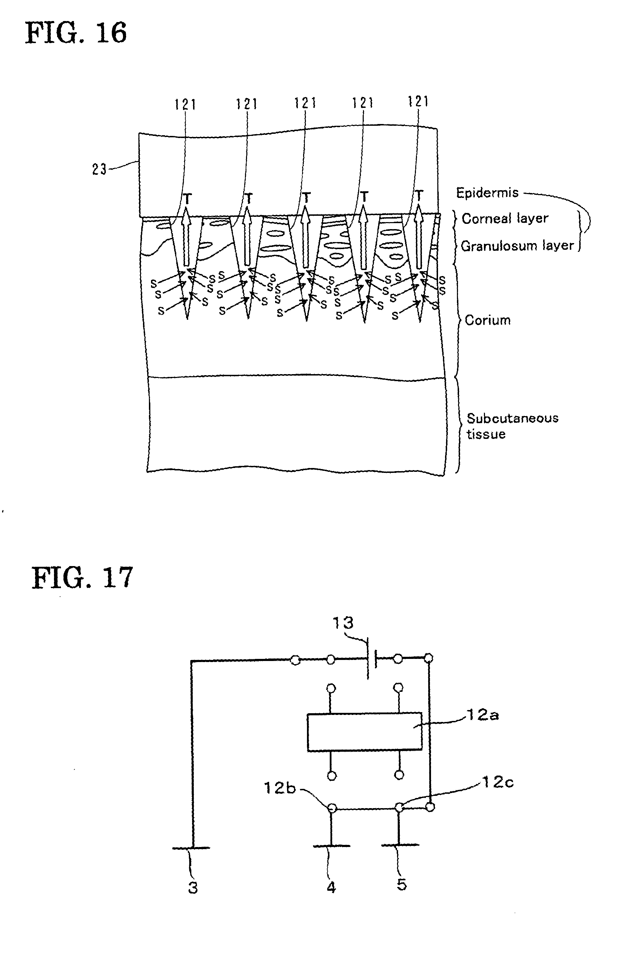

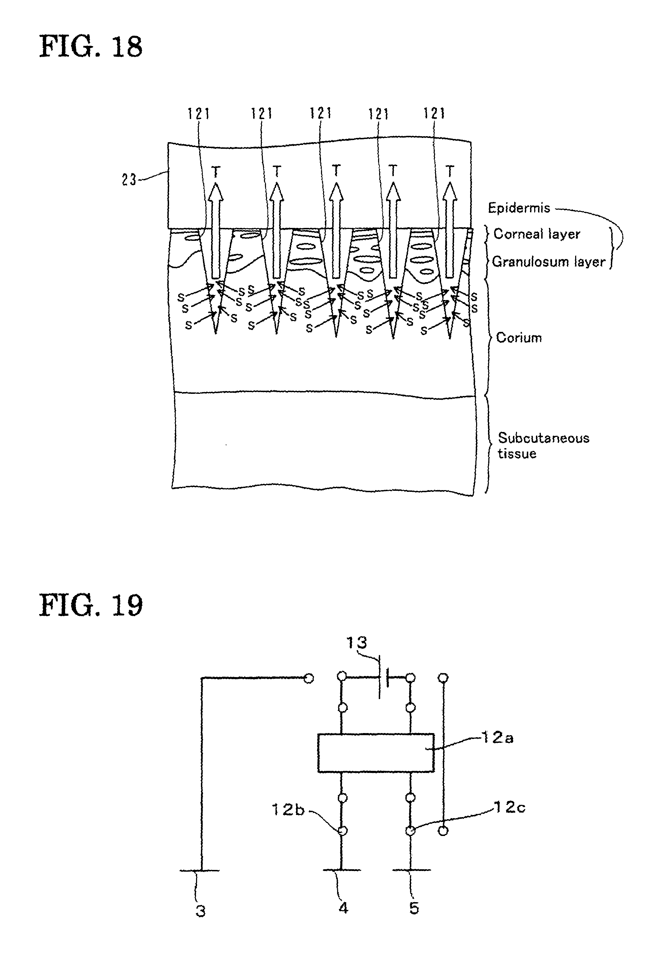

The switching circuit 12 is configured by an assembly of switching elements, and is provided for switching the output destination of the constant current power supplier 13. Two terminals 12b and 12c (refer to Figs. 17 and 19) are connected to the switching circuit 12, and the terminals 12b and 12c are provided on the surface of the analyzing unit 6 so as to contact with the electrodes 4 and 5 of the extraction cartridge 2 when the extraction cartridge 2 is fixed on the analyzing unit 6. Furthermore, the switching circuit 12 is electrically connected to the electrode 3 and the constant current voltage 13 (refer to Fig. 17). An inverter 12a is also connected to the switching circuit 12 to convert the direct current to an alternating current.

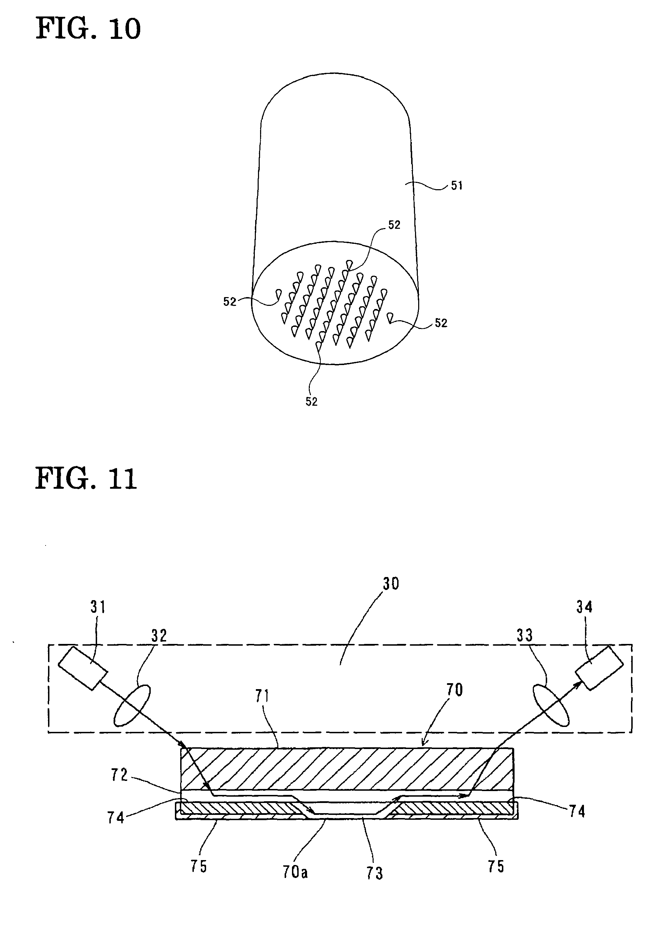

The detector 30 is provided to detect the amount of extracted glucose. As shown in Fig. 11, the detector 30 is configured by a monochrome light source 31, lens 32, lens 33, and photoreceptor 34. The monochrome light source 31 functions to emit light for analysis to a glucose sensor 70 via the lens 32. The light, which is emitted from the monochrome light source 31 through the lens 32 to the glucose sensor 70, passes through a predetermined path of the glucose sensor 70, and impinges the photoreceptor 34 through the lens 33. The photoreceptor 34 functions to output a signal based on the amount of received light.

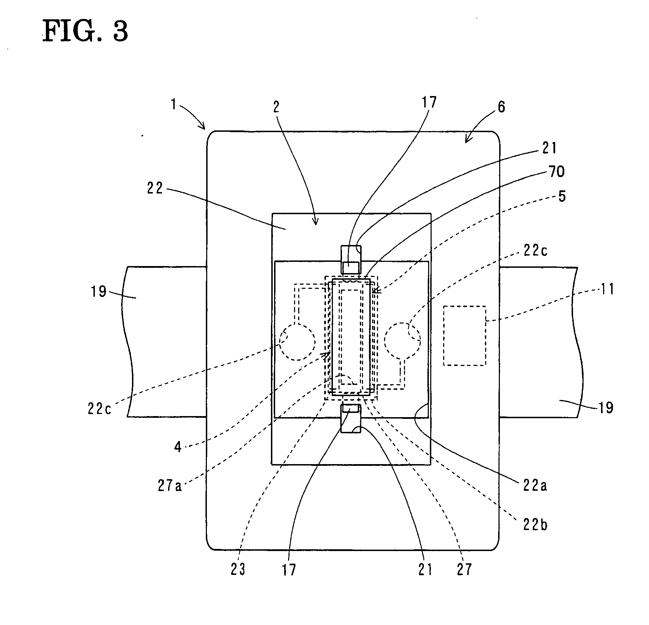

As shown in Fig. 5, the extraction cartridge 2 is provided with a cartridge body 22 formed of acrylic rubber, electrode 4, electrode 5, masking tape 27, mesh sheet 23, and glucose sensor 70. As shown in Fig. 3, the extraction cartridge 2 is removably fixed to the analyzing unit 6 by engaging each of the connector hooks 17 of the analyzing unit 6 with the two mounting holes 21 formed in the extraction cartridge 2. The extraction cartridge 2 is configured so as to be capable of replaced and used for each glucose measurement.

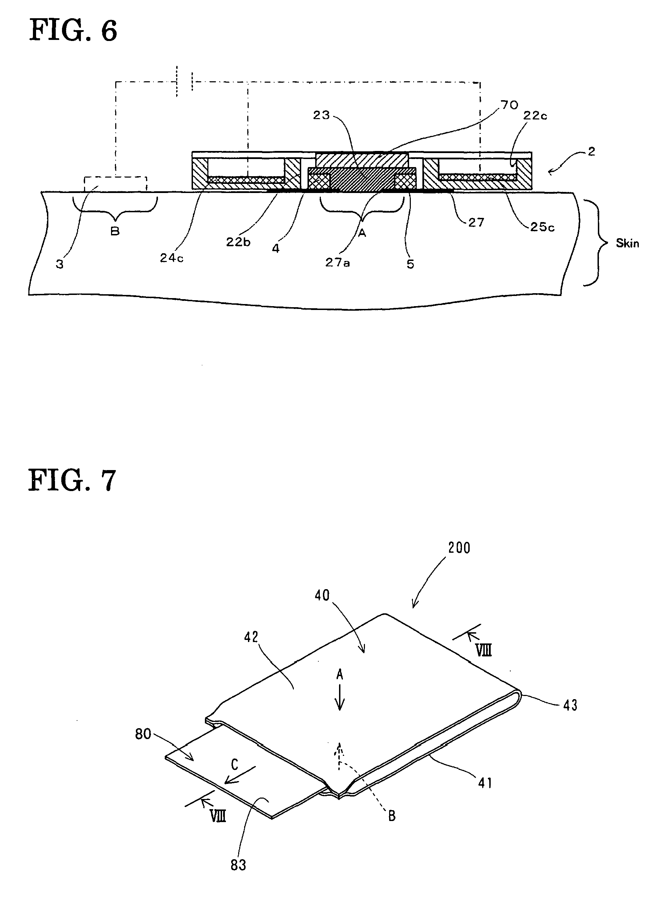

A square-shaped concave part 22a is provided on the extraction cartridge body 22, as shown in Fig. 5. As shown in Figs. 5 and 6, a through hole 22b that reaches to the bottom surface of the cartridge body 22 is formed in the center of the concave part 22a. Furthermore, a circular concavities 22c are respectively provided at opposed positions across the through holes 22b of the concave part 22a. The terminals 24c and 25c of the electrodes 4 and 5 are inserted in the two concavities 22c. The terminals 24c and 25c contact with the two terminals 12b and 12c on the analyzing unit 6 side connected to the switching circuit 12 when the extraction cartridge 2 is fixed on the analyzing unit 6, such that the electrodes 4 and 5 are electrically connected to the constant current power supplier 13.

Silver/silver chloride electrodes are used for the electrode. 4 and electrode 5.

The masking tape 27 is provided to prevent direct contact of the skin and the electrodes since the current is known to be painful to the skin. Therefore, the masking tape 27 is an insulating member. As shown in Fig. 6, the masking tape 27 contacts with the bottom surfaces of the electrode 4 and electrode 5. Furthermore, an opening 27a is provided in the masking tape 27, such that tissue fluid can be extracted from the extraction site of the skin to the extraction medium held by the mesh sheet 23 via the opening 27a.

The mesh sheet 23 is provided to hold the extraction medium for extracting tissue fluid. The mesh sheet 23 used in the present embodiment is formed of nylon, approximately 10 mm in length, approximately 4 mm in width, and approximately 50 $\mu$m in thickness. Purified water is used as the extraction medium, and the mesh sheet 23 is supplied with approximately 1.5 $\mu$m of purified water before measurement begins in the blood glucose level measuring device 1. The purified water used in the present embodiment has an electrical resistivity of 18.3 M$\Omega$, and is essentially an insulator (electrically non-conductive substance).

The mesh sheet 23 essentially lacks elasticity, and the thickness is substantially unchanged. Furthermore, the mesh sheet 23 is formed of braided nylon fiber approximately 30 $\mu$m in thickness, and is a square woven form, 33 $\mu$m by 33 $\mu$m. As shown in Fig. 6, the mesh sheet 23 contacts with the top surface of electrode 4 and electrode 5, and contacts with the bottom surface of the glucose sensor 70.

The glucose sensor 70 is provided to detect the glucose in the tissue fluid extracted to the extraction medium held by the mesh sheet 23. As shown in Fig. 11, the bottom surface of the glucose sensor 70 has a measuring surface 70a. On the measuring surface 70a is applied a color-producing agent to produces a color in reaction with active oxygen, an enzyme (peroxidase) as a catalyst for hydrogen peroxide, and an enzyme (glucose oxidase) as a catalyst for glucose. Furthermore, the glucose sensor 70 is configured by a glass substrate 71, first optical waveguide layer 72 mounted below the substrate 71, second optical waveguide layer 73 mounted below the first optical waveguide layer 72, protective layer 74 formed below the first optical waveguide layer 72 so as to sandwich the second optical waveguide layer 73 between, and light shield layer 75 covering the exterior side of the protective layer 74. The first optical waveguide layer 72 has a higher refractive index than the substrate 71. The second optical waveguide layer 73 has a trapezoidal shape inclined to the side, and has a higher refractive index than the first optical waveguide layer 72. The measurement layer 70a of the glucose sensor 70 is an exposed area from the protective layer 74 of the second optical waveguide layer 73, and contacts with the surface of the mesh sheet 23.

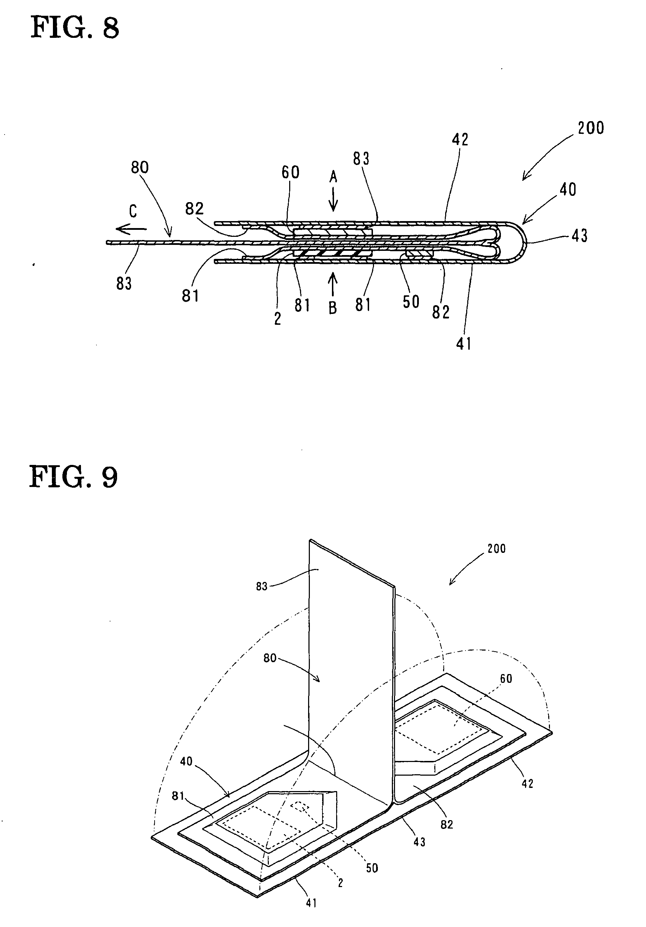

The structure of the extraction cartridge set 200 that houses the unused extraction cartridge 2 installed in the blood glucose level measuring device 1 is described below. The extraction cartridge set 200 is configured so as to house the unused extraction cartridge 2 installed in the blood glucose level measuring device 1 in a dry condition, and be capable of including a predetermined amount of purified water (approximately 1.5 $\mu$l in the present embodiment) for the extraction cartridge 2 when installed in the blood glucose level measuring device 1. The extraction cartridge set 200 is provided with a support member 40, the previously mentioned extraction cartridge 2, desiccant 50, liquid supplying member 60, and separating member 80, as shown in Fig. 8.

The support member 40 is sheet-like and flexible. The support member 40 is also curved in a U-shape, as shown in Fig. 7. As shown in Fig. 9, the support member 40 is configured by a cartridge support 41, liquid supplying member support 42 disposed opposite the cartridge support 41, and curved part 43 that links to the cartridge support 41 and the liquid supplying member support 42. Since the support member 40 is essentially shaped as a wallet, it is capable of holding (housing) the extraction cartridge 2 and the liquid supplying member 60 within the region circumscribed by the cartridge support 41, the liquid supplying member support 42 and the curved part 43.

The extraction cartridge 2 is installed so as to be easily removable in the interior side of the cartridge support 41 of the support member 40.

The desiccant 50 is provided to inhibit the mesh sheet 23 of the extraction cartridge 2 from absorbing atmospheric moisture and becoming wet.

The liquid supplying member 60 is a non-woven cloth of absorbent cotton (cut cotton) measuring approximately 15 mm in length, approximately 15 mm in width, and approximately 50 $\mu$m in thickness. The liquid supplying member 60 absorbs and holds a predetermined amount (150 $\mu$l in the present embodiment) of purified water. As shown in Fig. 8, the liquid supplying member 60 is fixedly attached top the interior surface of the liquid supplying member support 42 of the support member 40 so as to confront the mesh sheet 23 of the extraction cartridge 2 mounted on the cartridge support member 41 of the support member 40. In the present embodiment, approximately 1% of the purified water (approximately 1.5 $\mu$l) from the purified water (approximately 150 $\mu$l) absorbed by the liquid supplying member 60 is supplied from the liquid supplying member 60 to the mesh sheet 23 of the extraction cartridge 2.

The separating member 80 is sheet-like and flexible, similar to the support member 40 described above. The separating member 80 is housed in a region circumscribed by the cartridge support 41 of the U-shaped support member 40, liquid supplying member support 42, and curved part 43, as shown in Fig. 8. The separating member 80 is provided with a grip portion 83, which is provided to be gripped by a subject when the separating member 80 is removed from the support member 40. The cartridge housing part 81 and liquid supplying member housing part 82 can be gradually peeled from the cartridge support 41 and liquid supplying member support 42 of the support member 40 by gripping and pulling the grip portion 83 in the arrow C direction.

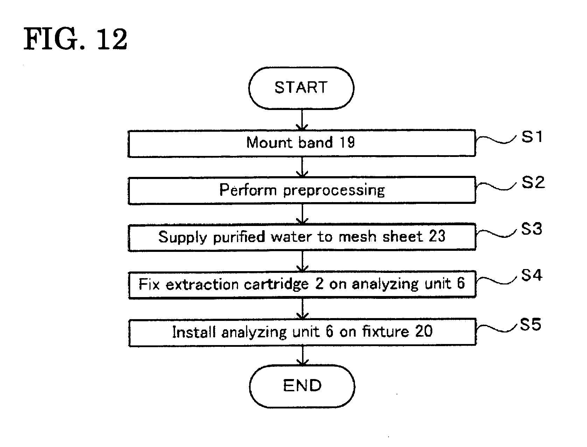

The subject preparation sequence for measurement by the blood glucose level measuring device 1 is described below using Fig. 12.

[Preparation Sequence]

The subject first mounts the band 19 on the wrist (step S1). At this time the band 19 is installed so that the extraction site A on the skin of the subject (refer to Fig. 6) is positioned within the opening 20a of the fixture 20 of the band 19 (refer to Fig. 2). Since the electrode 3 is provided on the back surface of the band 19, the electrode 3 contacts with the surface of the skin B (refer to Fig. 6) when the band is installed on the wrist.

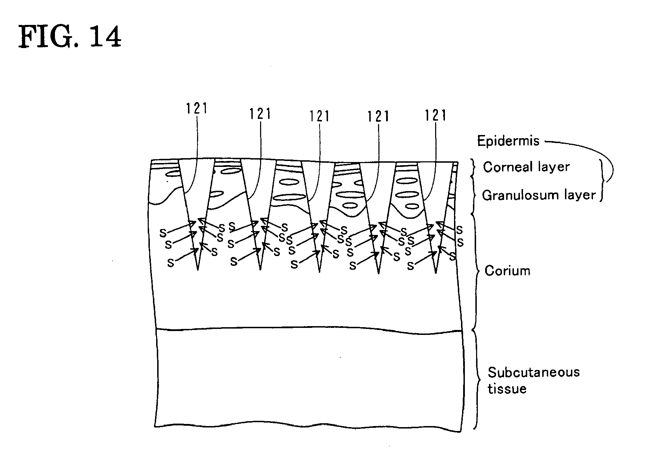

The subject then performs preprocessing of the extraction site A (refer to Fig. 6) on the wrist of the subject (step S2). Specifically, the subject pierces the extraction site A using the micro needle array 51 shown in Fig. 10. Forty-nine micro needles 52 having a length of 0.4 mm and thickness of 0.24 mm protrude at equal spacing within a $10 \times 10$ mm surface area on the leading end surface of the micro needle array 51. By means of the preprocessing, micro pores are formed in the epidermis at the extraction site A, such that tissue fluid can be readily extracted transdermally at the extraction site A. In the present embodiment, the extraction site A on the wrist of the subject is pierced through the opening 20a of the fixture 20 of the band 19 so as to accurately match the measuring location and the piercing location. A plurality of extraction holes 121 formed at the extraction site A in this step extend through the corneum layer and granulosum layer and reach near the middle of the corium, but do not reach the subcutaneous tissue. The extraction holes 121 are formed such that their diameter is greater at the skin surface and the diameter is smaller near the subcutaneous tissue. When the extraction holes 121 are formed, tissue fluid filling the corium spreads into the extraction holes 121, as indicated by the arrow S. Glucose is present in this tissue fluid. See Fig. 14.

The subject supplies purified water to the mesh sheet 23 in conjunction with the removal of the unused extraction cartridge 2 from the extraction cartridge set 200 (step S3). Specifically, the subject draws the grip portion 83 of the separating member 80 of the extraction cartridge set 200 in the arrow C direction as shown in Fig. 7. Thus, the separating member 80 is pulled from between the dry extraction cartridge 2 and the liquid supplying member 60 that contains purified water, and the cartridge support 41 and liquid supplying member support 42 of the support 40 are pushed from the arrow A direction and arrow B direction by the finger of the subject. In this way the liquid supplying member 60 which contains purified water comes into contact with the dry extraction cartridge 2, and approximately 1.5 μl of purified water held by the liquid supplying member 60 is supplied to the mesh sheet 23.

Next, the subject fixes the extraction cartridge 2 that contains the mesh sheet 23 that is saturated with a predetermined amount (approximately 1.5 μl) of purified water to the analyzing unit 6 by engaging the two connector hooks 17 of the analyzing unit 6 to the pair of mounting holes 21 of the extraction cartridge 2 (step S4).

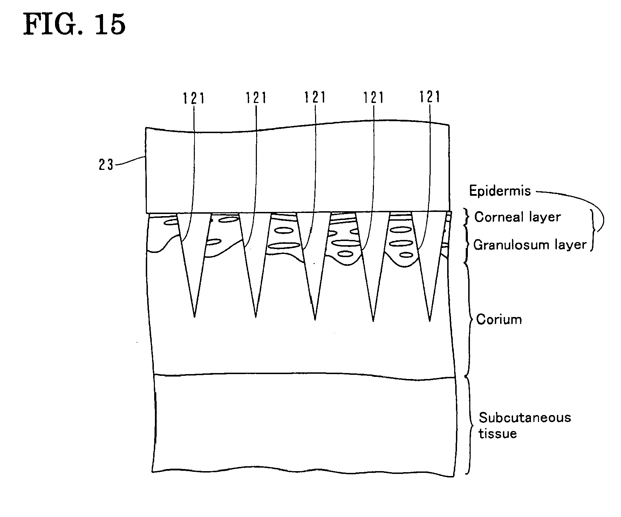

Thereafter, the subject installs the analyzing unit 6 with the fixed extraction cartridge 2 on the fixture 20 of the band 19 (step S5). In this way the blood glucose level measuring device 1 is mounted on the wrist of the subject with the purified water-saturated mesh sheet 23 in a state of contact with the skin extraction site A. Thus, the purified water flows from the mesh sheet 23 that is in contact with the extraction site A into the extraction holes 121, as shown in Fig. 15. When the purified water flows into the extraction holes 121, the tissue fluid that seeped into the extraction holes 121 due to the formation of the extraction holes 121 in step S1 migrates in the direction of the mesh sheet 23 (the T direction in Fig. 16) as shown in Fig. 16. Then, tissue fluid again flows from the corium to the extraction holes 121 because the osmotic pressure within the extraction hole 121 is lower than the osmotic pressure of the corium of the skin.

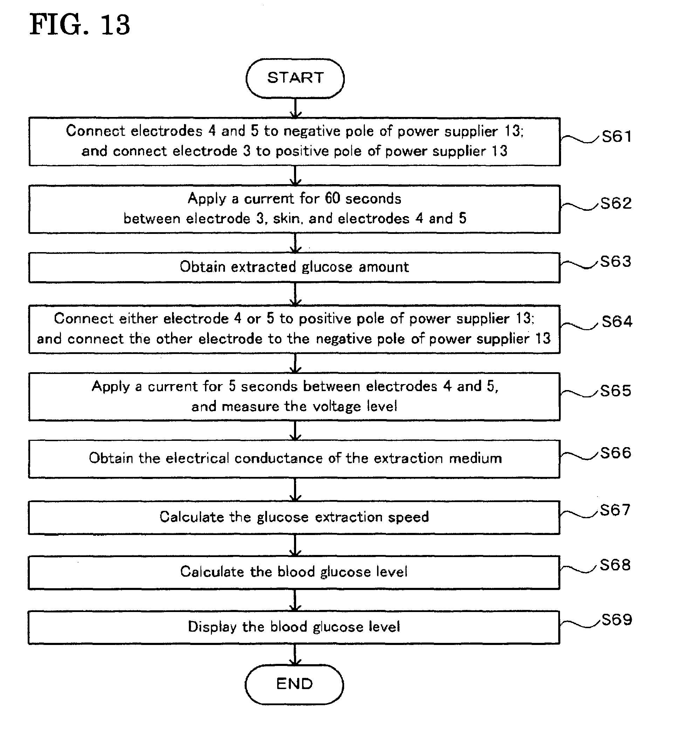

After this preparation, the subject measures the blood glucose level via the blood glucose level measuring device 1. Specifically, the subject operates the input part 14 to input a measurement start command to the controller-analyzer 11 of the blood glucose level measuring device 1, and the controller-analyzer 11 starts the measuring process. Fig. 13 is a flow chart showing the sequence of the measuring process performed by the controller-analyzer 11 of the blood glucose level measuring device 1. Each step of this process is described using the flow chart of Fig. 13.

Measuring Sequence of the Blood glucose Level Measuring Device 1

When the measuring start command from the input part 14 is input to the controller-analyzer 11, the controller-analyzer 11 starts the extraction process. Specifically, the controller-analyzer 11 controls the switching circuit 12 so as to electrically connect the electrode 4 and electrode 5 positioned in the vicinity of the extraction site A to the negative pole of the constant current power supplier 13, and connect the electrode 3 in contact with the skin surface B to the positive pole of the constant current power supplier 13, respectively. Fig. 17 shows the electrical circuit that results from the control performed in this step, and in this condition an electric field is applied to the skin of the subject.

Then the controller-analyzer 11 controls the constant current power supplier 13 such that a direct current of 150 μA is applied for a predetermined time T (60 seconds in the present embodiment) between the electrode 3, electrode 4, and electrode 5.

Since the tissue fluid that has seeped into the extraction holes 121 carries an electrical charge, the migration of the tissue fluid is accelerated toward the mesh sheet 23 (T direction in Fig. 18) by imparting an electrical field using the constant current power supplier 13, as shown in Fig. 18. Although the glucose contained in the tissue fluid does not carry an electrical charge, the glucose migrates in conjunction with the migration of other components that do carry an electrical charge. The glucose that has moved to the extraction medium within the mesh sheet 23 is diffused (so-called passive diffusion) within the extraction medium, and arrives at the glucose sensor 70. The glucose that reaches the measuring surface 70a reacts with the glucose oxidase catalyst, and the hydrogen peroxide that is generated as a result then reacts with the peroxidase catalyst. As a result, active oxygen is generated. The color-producing agent painted on the measuring surface 70a reacts with the active oxygen and a color is produced. Therefore, the color-producing agent produces a color at an intensity that corresponds to the amount of glucose extracted from the body.

Light, which is totally reflected within the second optical waveguide layer 73 of the glucose sensor 70 in contact with the mesh sheet 23 saturated with purified water, is absorbed by the color-producing agent in accordance with the amount of glucose extracted from the body, and thereafter arrives at the photoreceptor 34. As a result, the light, with intensity that is commensurate with the amount of glucose which has arrived at the measuring surface 70a, impinges the photoreceptor 34, and a signal corresponding to the intensity of the impinging light is output from the photoreceptor 34.

After a predetermined time has elapsed from the application of a current to the skin (60 seconds in the present embodiment), the controller-analyzer 11 obtains an extracted glucose amount (Q) based on the signals output from the photoreceptor 34 (step S63).

Subsequent to obtaining the extracted glucose amount (Q), the controller-analyzer 11 obtains the electrical conductance of the extraction medium. Specifically, the controller-analyzer 11 controls the switching circuit 12 so as to connect one of the electrodes 4 and 5 to the positive pole of the constant current power supplier 13, and connect the other electrode to the negative pole of the constant current power supplier 13 (step S64). In the present embodiment, an inverter 12a is provided in the switching circuit 12, so as to have a 50 Hz alternating current flow between the electrode 4 and electrode 5. Fig. 19 shows the electrical circuit that results from the control performed in step 64, and in this condition the electrical conductance of the extraction medium is measured.

Then, the controller-analyzer 11 controls the constant current power supplier 13 so that a constant current of 50 μA is applied for 5 seconds between the electrode 4 and electrode 5, and controls the voltmeter 16 so as to measure the voltage level (X) at this time.

Thereafter, the controller-analyzer 11 calculates the electrical resistance value (R) of the extraction medium as R=X/I by dividing the voltage level (X) by the current value (I) (50 μA in the present embodiment), and obtains the electrical conductance (K) of the extraction medium as K=1/R=I/X (step S66). The electrical conductance of the extraction medium is calculated as the reciprocal of the electrical resistance value (R).

Then, the controller-analyzer 11 calculates the glucose extraction speed (J) indicating the amount of glucose extracted per unit time (1 second in the present embodiment) (step S67). The glucose extraction speed (J) is calculated as J=Q/T by dividing the extracted glucose amount (Q) obtained in step S63 by the predetermined time T during which the constant current regulation power supplier 13 applied a current (60 seconds in the present embodiment).

Then the controller-analyzer 11 calculates the blood glucose level using the equation (1) below based on the electrical conductance (K) of the extraction medium obtained in step S66, and the glucose extraction speed (J) obtained in step S67 (step S68). Although the value calculated using equation (1) below is the glucose concentration in the tissue fluid of the body, the calculation result of equation (1) is used as the blood glucose level in the present embodiment since the glucose concentration of the tissue fluid and the glucose concentration of the blood (that is, the blood glucose level) are virtually equal.

$$BG = J/P$$
$$= J/(ak+b) \cdot \cdot \cdot (1)$$

In equation (1), "BG" represents the calculated blood glucose level, "J" represents the glucose extraction speed which is the amount of glucose extracted per unit time, "P" represents the glucose permeability (ease of glucose pass-through) at the extraction site A, and "K" represents the electrical conductance calculated in step S63. Furthermore, "a" and "b" represent preset constants. Equation (1) is stored in the controller-analyzer 11 beforehand and is read therefrom to calculate the blood glucose level each time the blood glucose level is measured. The principle for the blood glucose level calculation used in the present embodiment is described below.

The controller-analyzer 11 performs control so as to display the blood glucose level calculated in step S68 on the display 15 (step S69). By displaying the blood glucose level on the display 15, the subject can thus know his own blood glucose level without the need to collect blood.

[Principle of the Calculation of the Blood glucose Level]

The principle for the blood glucose level calculation used in the present embodiment is described below.

When a blood glucose level is calculated from the amount of extracted glucose as in the present embodiment, correcting the extracted glucose amount by the glucose permeability (P) of the skin provides a more precise blood glucose level calculation because skin conditions differ by subject and the extracted glucose amount changes depending on the condition of the skin. For example, when the skin is pierced using the same micro needle array 51, the amount of extracted glucose may increase if the subject has a thin and soft corneum layer because the micro pores are easily formed. On the other hand, the amount of extracted glucose may decrease when the subject has a hard and thick corneum layer because the micro pores are more difficult to be formed. Therefore, in the present embodiment, the glucose permeability (P) at the extraction site A is estimated, and the blood glucose level is calculated by the equation (BG=J/P).

The electrolyte concentration in the tissue fluid is known to be substantially the same among a plurality of subjects with different blood glucose level. Therefore, it is possible to estimate the degree of the tissue fluid permeation through the skin (that is, the glucose permeability (P)) by measuring the electrolyte content contained in the tissue fluid extracted transdermally. In the present embodiment, purified water that does not contain electrolyte is used as an extraction medium for holding the extracted tissue fluid, and the amount of electrolyte contained in the extracted tissue fluid is estimated by measuring the electrical conductance (K) of the extraction medium when electric power is supplied to the extraction medium containing the extracted tissue fluid. That is, the glucose permeability (P) is estimated from the electrical conductance (K) of the extraction medium containing the extracted tissue fluid.

Equation (1) is determined by the following experiment. First, a plurality of subjects with a blood glucose level (t) (a constant) (blood glucose level: 80 in the present embodiment) are repeatedly subjected to identical operations of steps S1 to S5, and Step S6 to S66. In this way a plurality of glucose extraction speeds (J) and electrical conductances (K) of the extraction medium are obtained.

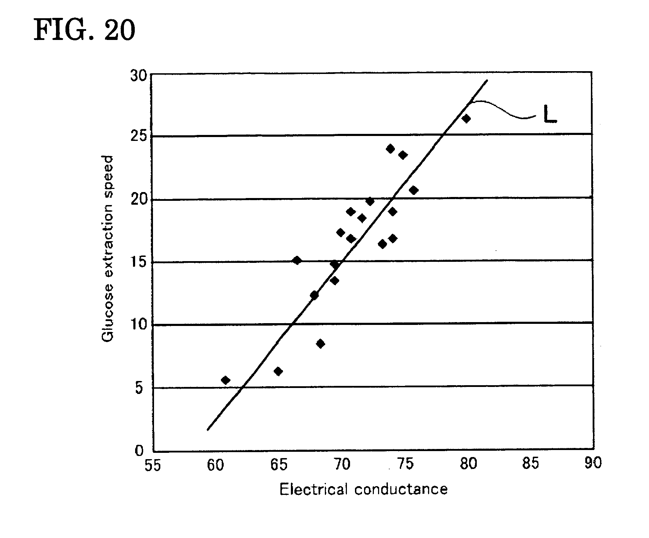

Then, a plurality of coordinate data (J,K), which are derived from the obtained glucose extraction speed (J) and electrical conductance (K) of the extraction medium, are plotted with the glucose extraction speed (J) on the vertical axis and the electrical conductance (K) of the extraction medium on the horizontal axis. The characteristics chart shown in Fig. 20 is thus obtained. It can be understood from the characteristics chart that the plotted glucose extraction speed (J) and electrical conductance (K) of the extraction medium have a proportional relationship. Thereafter, a linear regression L which represents the relationship between the plotted glucose extraction speed (J) and electrical conductance (K) of the extraction medium, is drawn on the characteristics chart, and the equation (J=$\alpha$K+$\beta$) which represents the line L is determined.

The relation (J=$\alpha$K+$\beta$) which represents the line L is thus established between glucose extraction speed (J) and electrical conductance (K) of the extraction medium obtained from subjects who have a blood glucose level (t) (constant) (blood glucose level: 80 in the present embodiment). The expression "t=($\alpha$K+($\beta$))/P" is obtained by substituting "$\alpha$K+$\beta$" for "J" and "t" for "BG" in the expression (BG=J/P) which is a premise of equation (1).

The glucose permeability (P) can thus be determined as P=($\alpha$K+$\beta$)/t from the above expression.

Then, BG=tJ/($\alpha$K+$\beta$)=J/(aK+b) (where a=$\alpha$/t, and b=$\beta$/t) of equation (1) is obtained by substituting "($\alpha$K+$\beta$)/t" for "P" in the expression (BG=J/P) which is a premise of equation (1).

Therefore, discomfort of the subject can be mitigated since the present invention provides an analyzer (measuring device) and analyzing method (measuring method) for obtaining the concentration of predetermined components contained in the tissue fluid in the body without collecting blood.

Although the tissue fluid is extracted by a method in which a current flows to the extraction site in the present embodiment, tissue fluid may also be extracted without a current flow (so-called passive diffusion extraction).

Although the tissue fluid is extracted after preprocessing using the micro needle array 51 in the present embodiment, the blood glucose level measuring device 1 may performs the measurements without preprocessing.

Although the glucose extraction speed is used to determine the blood glucose level in the present embodiment, the glucose concentration in the extraction medium held by the mesh sheet 23 may also be used.

Although the glucose concentration in the tissue fluid of the body of the subject is considered as the blood glucose level in the present embodiment, a correction may also be performed to convert the glucose concentration in the tissue fluid of the body of the subject to a blood glucose level.

Although the mesh sheet 23 is dry before the measurement in the present embodiment, a predetermined amount of purified water may be held by the mesh sheet 23 beforehand. In this case, if the purified water held in the mesh sheet 23 has evaporated, a predetermined amount of purified water can be held by the mesh sheet 23 by supplying purified water to the mesh sheet 23 when measurement starts.

Although the extraction cartridge 2 is installed in the analyzing unit 6 after purified water has been supplied to the mesh sheet 23 provided in the extraction cartridge 2 in the present embodiment, purified water may also be supplied to the mesh sheet 23 after the extraction cartridge 2 is installed in the analyzing unit 6. This configuration can reduce a deterioration on the glucose sensor 70 caused by contact with the purified water and the extraction cartridge 2.

In the present embodiment, the distance between the skin and the glucose sensor during measurement can be uniformly maintained with ease by using a mesh sheet 23 formed of nylon, because the thickness of the mesh sheet 23 doesn't change when the extraction cartridge 2 is pressed against the skin of the subject. Therefore, measurement errors are reduced.

Although the extraction cartridge 2 is provided with a mesh sheet 23 in the extraction medium holder formed in a space between the masking tape 27 and the glucose sensor 70 in the present embodiment, the extraction medium holder may also be provided with an absorbent member such as a piece of paper for absorbing and retaining a fluid as a member for holding the extraction medium fluid. Moreover, the extraction medium holder need not be provided with a member such as the mesh sheet 23 and the like, if a space is provided to hold the extraction medium. An electrically non-conductive gel may also be used as the extraction medium held in the space.

Although purified water is used as the extraction medium supplied to the mesh sheet 23 in the present embodiment, an electrically non-conductive fluid other than purified water may be supplied to the mesh sheet 23.

Although the glucose permeability is estimated by extracting tissue fluid into purified water that does not contain electrolyte and by measuring the electrical conductance of the extraction medium containing the extracted tissue fluid in the present embodiment, glucose permeability may also be estimated by extracting tissue fluid to an electrolytic solution such as physiological saline or the like. Note that use of an electrically non-conductive extraction medium is desirable to estimate the glucose permeability with greater precision.

Although a direct current flow to the skin is provided using a DC constant current power supplier 13 in the present embodiment, a direct current flow to the skin may also be provided using an AC constant current power supplier by providing in the circuit a converter for converting the alternating current to a direct current.

Although glucose is transdermally extracted by applying a constant current from a constant current power supplier to the skin of a subject and the amount of extracted glucose is corrected using the electrical conductance of the extraction medium in the present embodiment, glucose may also be extracted transdermally by applying a constant voltage from a constant voltage power supplier. Note that, when extracting glucose using an application of a constant voltage, the magnitude of the current flowing to the skin changes according to the electrical resistance of the skin. Since the amount of extracted glucose is dependent on the magnitude of the current flowing to the skin, increasing the current value will increase the amount of extracted glucose. Therefore, when glucose is extracted using a constant voltage, a current measuring section may be provided to monitor the change in the current value during glucose extraction and the amount of extracted glucose may be corrected based on the current value monitored by the current measuring section, in addition to the correction of the extracted glucose amount using the electrical conductance of the extraction medium. For example, the amount of extracted glucose may also be corrected by monitoring the change in the current value during glucose extraction, calculating an average current value, and determining a ratio between the average current value and a standard current value, in addition to the correction using the electrical conductance of the extraction medium.

Although the electrical conductance of the extraction medium is measured when electric power is supplied to the extraction medium holding tissue fluid extracted transdermally in order to estimate glucose permeability in the present embodiment, the electrical resistance value of the extraction medium when electric power is supplied to the extraction medium, the voltage level applied to the extraction medium, and the current value applied to the extraction medium may be measured. It is also possible to estimate glucose permeability based on the magnitude of the electrical resistance, voltage, and current.

Although electrical information such as the electrical conductance of the extraction medium is obtained to estimate glucose permeability in the present embodiment, information may also be obtained that reflects the degree of glucose permeation through the skin by measuring the amount of ions present in the tissue fluid extracted transdermally using ion electrodes, insofar as the information obtained reflects the skin permeability of glucose.

Although silver/silver chloride electrodes are used as the electrode 4 and electrode 5 in the present embodiment, activated carbon electrodes may also be used. When activated carbon electrodes are used, the correlation between the glucose extraction speed and the electrical conductance of the extraction medium can be obtained with greater precision.

In the present embodiment, the electrodes 4 and 5 are used both when a current flows to the extraction medium and when an electric field is applied to the skin of the subject. This configuration reduces the number of electrodes used in the blood glucose level measuring device 1 and renders the device more compact. In the present embodiment, a switching circuit 12 is provided to switch the output destination of the constant current power supplier 13. Thus, the number of power suppliers can be reduced, and the device can be rendered even more compact.

Although the electrodes 4 and 5 are used both when a current flows to the extraction medium and when an electric field is applied to the skin of the subject in the current embodiment, an electrode other than the electrodes 3, 4, and 5 may also be provided independently such that the electrodes 4 and 5 are used when a current flows to the extraction medium, and a current flows between the electrode 3 and the independently provided electrode when an electric field is applied to the skin of the subject.

Although the blood glucose level of the subject is displayed on the display 15 in the present embodiment, electrical information obtained by supplying electrical power to the extraction medium, such as the electrical conductance and electrical resistance value of the extraction medium, also may be displayed on the display 15. Accordingly, the subject easily obtains information reflecting the glucose permeability of the skin.

Although a blood glucose level measuring device 1 for measuring a blood glucose level has been described as an embodiment of the analyzer in the present embodiment, the embodiment is also applicable to analyzers that obtain the concentrations of components in the tissue fluid in the body of the subject based on analysis values of the components contained in the tissue fluid extracted from the body of the subject. The analyzer of the present invention may also measure the concentration of analyzable components such as protein as a biochemical component, the concentrations of drugs administered to the subject and the like. These components may be extracted into the extraction medium in the same manner as in the present embodiment.

**Claims**

1. An analyzer, comprising:

   an extraction medium retainer for retaining an extraction medium for holding tissue fluid extracted through a skin of a subject;
   a component amount information obtainer for obtaining component amount information regarding amount of a predetermined component contained in the tissue fluid held in the extraction medium;
   an electrical information obtainer for obtaining electrical information related to amount of the extracted tissue fluid by supplying electrical power to the extraction medium which is holding the extracted tissue fluid; and
   a component concentration obtainer for obtaining a concentration of the predetermined component contained in body fluid of the subject based on the component amount information and the electrical information.

2. The analyzer of claim 1, wherein
   the body fluid is selected from tissue fluid and blood.

3. The analyzer of claim 1 or claim 2, wherein
   the electrical information reflects the degree of tissue fluid permeation through the skin.

4. The analyzer of any one of claim 1 to 3, wherein
   the electrical information relates to electrical conductance or electrical resistance of the extraction medium that is holding extracted tissue fluid.

5. The analyzer of any one of claim 1 to 4, wherein
   the electrical information relates to an electrical current or a voltage applied to the extraction medium holding the extracted tissue fluid.

6. The analyzer of any one of claim 1 to 5,
   wherein the electrical information obtainer comprises a first electrode, a second electrode, and an electrical power supplier for applying an electrical current between the first electrode and the second electrode, and wherein the first electrode and the second electrode are disposed so as to contact with the extraction medium retained by the extraction medium retainer.

7. The analyzer of claim 6,
   wherein the electrical information obtainer further comprises a third electrode electrically connected to the electrical power supplier,
   wherein the third electrode and at least one of the first electrode and second electrode are electrically connected to the skin of the subject when the analyzer is attached to the skin of the subject,
   and wherein the electrical power supplier applies an electrical current between the third electrode and the at least one of the first electrode and second electrode to extract the tissue fluid into the extraction medium.

8. The analyzer of claim 7,
   wherein the electrical information obtainer obtains a second electrical information by applying an electrical current between the third electrode and at least one of the first electrode and second electrode,
   and wherein the component concentration obtainer obtains the concentration of the predetermined component contained in the body fluid of the subject based on the second electrical information, the electrical information, and the component amount information.

9. The analyzer of any one of claim 1 to 8, wherein
   the extraction medium retained by the extraction medium retainer is non-conductive.

10. The analyzer of claim 9, wherein
    the extraction medium is water.

11. The analyzer of any one of claim 1 to 10, wherein
    the component amount information indicates amount of the predetermined component extracted into the extraction medium per unit time.

12. The analyzer of any one of claim 1 to 11, wherein
    the predetermined component is glucose.

**13.** The analyzer of claim 12, wherein
the concentration of the predetermined component contained in the body fluid of the subject is a blood glucose level.

**14.** The analyzer of any one of claim 1 to 13, further comprising
a display for displaying the electrical information.

**15.** An analyzing method, comprising steps of

(a) extracting tissue fluid through the skin of a subject into an extraction medium for holding the extracted tissue fluid;
(b) obtaining component amount information regarding amount of a predetermined component contained in the tissue fluid held in the extraction medium;
(c) obtaining electrical information related to amount of the extracted tissue fluid by supplying electrical power to the extraction medium which is holding the extracted tissue fluid; and
(d) obtaining a concentration of the predetermined component contained in body fluid of the subject based on the component amount information and the electrical information.

# FIG. 1

FIG. 2

# FIG. 3

# FIG. 4

# FIG. 5

# FIG. 6

# FIG. 7

# FIG. 8

# FIG. 9

## FIG. 10

## FIG. 11

# FIG. 12

```
          ┌─────────────┐
          │    START    │
          └──────┬──────┘
                 ▼
    ┌────────────────────────────┐
    │        Mount band 19       │ ─── S1
    └──────────────┬─────────────┘
                   ▼
    ┌────────────────────────────┐
    │     Perform preprocessing  │ ─── S2
    └──────────────┬─────────────┘
                   ▼
    ┌────────────────────────────────┐
    │ Supply purified water to mesh sheet 23 │ ─── S3
    └──────────────┬─────────────────┘
                   ▼
  ┌──────────────────────────────────────┐
  │ Fix extraction cartridge 2 on analyzing unit 6 │ ─── S4
  └──────────────┬───────────────────────┘
                 ▼
  ┌──────────────────────────────────────┐
  │  Install analyzing unit 6 on fixture 20 │ ─── S5
  └──────────────┬───────────────────────┘
                 ▼
          ┌─────────────┐
          │     END     │
          └─────────────┘
```

# FIG. 13

```
          ( START )
              │
              ▼
┌─────────────────────────────────────────────────────────┐
│ Connect electrodes 4 and 5 to negative pole of power     │      S61
│ supplier 13;                                             │
│ and connect electrode 3 to positive pole of power        │
│ supplier 13                                              │
└─────────────────────────────────────────────────────────┘
              │
              ▼
┌─────────────────────────────────────────────────────────┐
│ Apply a current for 60 seconds                           │      S62
│ between electrode 3, skin, and electrodes 4 and 5        │
└─────────────────────────────────────────────────────────┘
              │
              ▼
┌─────────────────────────────────────────────────────────┐
│ Obtain extracted glucose amount                          │      S63
└─────────────────────────────────────────────────────────┘
              │
              ▼
┌─────────────────────────────────────────────────────────┐
│ Connect either electrode 4 or 5 to positive pole of      │      S64
│ power supplier 13;                                       │
│ and connect the other electrode to the negative pole of  │
│ power supplier 13                                        │
└─────────────────────────────────────────────────────────┘
              │
              ▼
┌─────────────────────────────────────────────────────────┐
│ Apply a current for 5 seconds between electrodes 4 and 5,│      S65
│ and measure the voltage level                            │
└─────────────────────────────────────────────────────────┘
              │
              ▼
┌─────────────────────────────────────────────────────────┐
│ Obtain the electrical conductance of the extraction      │      S66
│ medium                                                   │
└─────────────────────────────────────────────────────────┘
              │
              ▼
┌─────────────────────────────────────────────────────────┐
│ Calculate the glucose extraction speed                   │      S67
└─────────────────────────────────────────────────────────┘
              │
              ▼
┌─────────────────────────────────────────────────────────┐
│ Calculate the blood glucose level                        │      S68
└─────────────────────────────────────────────────────────┘
              │
              ▼
┌─────────────────────────────────────────────────────────┐
│ Display the blood glucose level                          │      S69
└─────────────────────────────────────────────────────────┘
              │
              ▼
          (  END  )
```

FIG. 14

# FIG. 15

# FIG. 16

# FIG. 17

# FIG. 18

Epidermis — Corneal layer — Granulosum layer

Corium

Subcutaneous tissue

# FIG. 19

## FIG. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6607543 B

- WO 9600110 A